(19) Europäisches Patentamt
European Patent Office
Office européen des brevets

(11) **EP 2 844 336 B1**

(12) **EUROPEAN PATENT SPECIFICATION**

(45) Date of publication and mention
of the grant of the patent:
**31.05.2017 Bulletin 2017/22**

(51) Int Cl.:
***A61N 1/08*** (2006.01)

(21) Application number: **13708297.0**

(22) Date of filing: **01.03.2013**

(86) International application number:
**PCT/US2013/026622**

(87) International publication number:
**WO 2013/162681 (31.10.2013 Gazette 2013/44)**

(54) **ELECTRODE SELECTION BASED ON CURRENT SOURCE DENSITY ANALYSIS**

ELEKTRODENAUSWAHL AUF DER BASIS EINER STROMQUELLENDICHTEANALYSE

SÉLECTION D'ÉLECTRODE SUR LA BASE DE L'ANALYSE DE DENSITÉ DE SOURCE DE COURANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.04.2012 US 201261637011 P**
**18.02.2013 US 201313769526**

(43) Date of publication of application:
**11.03.2015 Bulletin 2015/11**

(73) Proprietor: **Medtronic, Inc.**
**Minneapolis, Minnesota 55432 (US)**

(72) Inventor: **MOLNAR, Gabriela C.**
**Minneapolis, MN 55432 (US)**

(74) Representative: **Hughes, Andrea Michelle**
**Dehns**
**St Bride's House**
**10 Salisbury Square**
**London EC4Y 8JD (GB)**

(56) References cited:
**WO-A2-2010/044989     WO-A2-2010/044989**
**US-A1- 2011 264 165**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD**

The disclosure relates to electrical stimulation, and, more particularly, to the selection of electrodes for use in delivering electrical stimulation. The invention is set out in the appended claims.

**BACKGROUND**

Implantable or external electrical stimulators deliver electrical therapy to a target tissue site within a patient with the aid of one or more electrodes, which may be deployed by medical leads. Electrical stimulators may be used in different therapeutic applications, such as deep brain stimulation (DBS), spinal cord stimulation (SCS), pelvic stimulation, gastric stimulation, peripheral nerve stimulation (PNS), and functional electrical stimulation (FES). Electrical stimulation may be used to deliver therapy to a patient to treat a variety of symptoms or patient conditions such as chronic pain, tremor, Parkinson's disease, other types of movement disorders, seizure disorders (e.g., epilepsy), urinary or fecal incontinence, sexual dysfunction, obesity, or psychiatric disorders. US-A-2011/0264165 and WO-A-2010/044989 describe selecting stimulation electrodes based on on frequency band characteristics of sensed physiological signals.

**SUMMARY**

An electrical stimulation device of the present disclosure senses electrical physiological activity of a patient using a plurality of electrodes. The stimulation device may determine a plurality of current source density (CSD) values based on the sensed electrical physiological activity. Each CSD value may be associated with a different electrode. Each CSD value may indicate the presence of one of a current source or a current sink in proximity to an associated electrode. The electrical stimulation device may control the delivery of electrical stimulation to the patient based on the determined CSD values.

In some examples according to the present disclosure, a system comprises a stimulation generator, a sensing module, and a processing module. The stimulation generator is configured to generate electrical stimulation. The sensing module is configured to sense electrical physiological signals generated by a patient via a plurality of electrodes. The processing module is configured to determine a plurality of CSD values based on the sensed electrical physiological signals. Each CSD value is associated with a different one of the plurality of electrodes. Each CSD value indicates the presence of one of a current source or a current sink in proximity to the electrode with which the CSD value is associated. The processing module is further configured to control the delivery of the electrical stimulation to the patient based on the CSD values.

In some examples according to the present disclosure, a method comprises sensing electrical physiological signals generated by a patient via a plurality of electrodes and determining a plurality of CSD values based on the sensed electrical physiological signals. Each CSD value is associated with a different one of the plurality of electrodes. Each CSD value indicates the presence of one of a current source or a current sink in proximity to the electrode with which the CSD value is associated. The method further comprises controlling delivery of electrical stimulation to the patient based on the CSD values.

In some examples according to the present disclosure, a system comprises means for sensing electrical physiological signals generated by a patient via a plurality of electrodes and means for determining a plurality of CSD values based on the sensed electrical physiological signals. Each CSD value is associated with a different one of the plurality of electrodes. Each CSD value indicates the presence of one of a current source or a current sink in proximity to the electrode with which the CSD value is associated. The system further comprises means for controlling delivery of electrical stimulation to the patient based on the CSD values.

In some examples according to the present disclosure, a system comprises a stimulation generator, a sensing module, and a processing module. The stimulation generator is configured to generate electrical stimulation. The sensing module is configured to sense electrical physiological signals generated by a patient via a plurality of electrodes. The processing module is configured to determine a power value for each of the plurality of electrodes, the power value indicating the power of the electrical physiological signals within a frequency band. The processing module is further configured to determine second differences of the power values for the plurality of electrodes and control the delivery of the electrical stimulation to the patient based on the second differences of the power values.

In some examples according to the present disclosure, a method comprises generating electrical stimulation and sensing electrical physiological signals generated by a patient via a plurality of electrodes. The method further comprises determining a power value for each of the plurality of electrodes, the power value indicating the power of the electrical physiological signals within a frequency band. Additionally, the method comprises determining second differences of the power values for the plurality of electrodes and controlling the delivery of the electrical stimulation to the patient based on the second differences of the power values.

In some examples according to the present disclosure, a system comprises means for generating electrical stimulation and means for sensing electrical physiological signals generated by a patient via a plurality of electrodes. The system further comprises means for determining a power value for each of the plurality of electrodes, the power value indicating the power of the electrical physiological signals within a frequency band. Additionally, the system comprises means for determining second differences of the power values for the plurality of electrodes and means for controlling the delivery of the electrical stimulation to the patient based on the second differences of the power values.

In other examples, a non-transitory storage medium stores instructions to cause a processor to receive an indication of sensed electrical physiological signals sensed from a patient via a plurality of electrodes, determine a power value for each of the plurality of electrodes, the power value indicating the power of the electrical physiological signals within a frequency band, determine second differences of the power values for the plurality of electrodes, and control the delivery of the electrical stimulation to the patient based on the second differences of the power values.

The details of one or more examples of the disclosure are set forth in the accompanying drawings and the description below. Other features, objects, and advantages will be apparent from the description and drawings, and from the claims. The present invention is set out in the appended claims. The embodiments, aspects or examples according to the present description that do not fall within the scope of said claims are provided for illustrative purposes only and do not form part of the present invention.

## BRIEF DESCRIPTION OF DRAWINGS

FIG. 1 is a conceptual diagram illustrating an example therapy system in the form of a deep brain stimulation system.

FIG. 2 is a functional block diagram illustrating components of an example implantable medical device.

FIG. 3 shows example electrode voltages, voltage differences, and current source density (CSD) values for electrodes along a lead.

FIG. 4 shows an example of a linear lead having N electrodes.

FIGS. 5A-5B show top and side views, respectively, of a linear lead having segmented electrodes.

FIGS. 6A-6C illustrate example electrodes arranged along a paddle lead.

FIG. 7 is a flow diagram illustrating an example method for selecting stimulation electrodes based on CSD values associated with the electrodes.

FIGS. 8A-8B show simulated monopolar potential measurements and simulated CSD value determinations.

FIGS. 9A-9B show simulated monopolar potential measurements, simulated CSD value determinations, and simulated voltage differences.

FIG. 10 is a flow diagram illustrating an example method for selecting stimulation electrodes based on second difference values associated with the electrodes.

## DETAILED DESCRIPTION

An electrical stimulation device of the present disclosure may select stimulation electrodes based on a current source density (CSD) value associated with the electrodes. As described herein, a CSD value may indicate the location and relative magnitude of current sources and current sinks in patient tissue (e.g., extracellular environment) surrounding the electrodes. The current sources and sinks may arise due to intrinsic neuronal activity of groups of neurons in the vicinity of (i.e., in proximity to) the electrodes. The electrical stimulation device of the present disclosure may sense the extracellular potentials, and use CSD analysis techniques to determine the locations of current sources and sinks relative to the electrodes along a lead. The electrical stimulation device may then control the delivery of electrical stimulation to the patient based on the CSD values associated with the electrodes.

In some examples, to determine a CSD value for an electrode, the stimulation device may first measure voltages associated with each of the electrodes, e.g., voltages generated by polarization of neurons in proximity to the electrode. The stimulation device may then determine a CSD value associated with the electrodes based on the measured voltages. In some examples, the stimulation device may determine the CSD values for the electrodes by determining a second order spatial difference of the voltages along the lead. Example equations which may be implemented by the stimulation device are described herein.

An electrical stimulation device of the present disclosure may include a sensing module and a stimulation generator. The sensing module senses electrical physiological activity of the patient using a plurality of electrodes. The stimulation generator delivers electrical stimulation to the patient using the plurality of electrodes. The stimulation device may also include a switching module that may be configured to connect either the stimulation generator to the plurality of electrodes or the sensing module to the plurality of electrodes. Accordingly, the stimulation device may be configured to provide electrical stimulation to a patient and/or sense electrical activity of the patient via a plurality of electrodes.

The stimulation device may include a processing module that controls the stimulation generator. The stimulation generator may include an adjustable voltage source and/or an adjustable current source(s) that generates the electrical stimulation to be delivered to the patient. In examples where the stimulation generator includes an adjustable voltage source, the processing module may control the adjustable voltage source to control the magnitude and polarity of the voltage delivered to the patient. In examples where the stimulation generator includes an adjustable current source, the processing module may control the adjustable current source to control the magnitude and direction of current delivered to the patient.

The processing module may control the switching module to selectively connect the stimulation generator and/or the sensing module to the plurality of electrodes. As described herein, the processing module may configure the switching module to connect the stimulation generator to the electrodes in order to deliver electrical stimulation to the patient. The processing module may also configure the switching module to connect the sensing module to the electrodes in order to sense electrical activity of the patient.

The sensing module may sense electrical physiological activity of the patient when the sensing module is connected to the electrodes via the switching module. For example, the sensing module may perform signal conditioning on the electrical physiological signals acquired via the electrodes. In some examples, the sensing module may amplify and filter the electrical signals received via the electrodes. The sensing module may also digitize the sensed electrical signals to generate sensing data. The sensing data may be digital data that indicates voltages associated with the electrodes.

The processing module may receive the sensing data from the sensing module and determine voltages associated with the plurality of electrodes based on the sensing data. For example, the processing module may determine a voltage associated with each of the electrodes. The voltage associated with an electrode may be referenced to any other electrode on the lead, or other electrode, such as a housing electrode on the stimulation device. Based on the sensing data generated by the sensing module, the processing module may determine a voltage difference between any two of the plurality of electrodes.

In systems including N electrodes, the electrodes may be referred to as a first electrode, a second electrode, ..., and an Nth electrode. The voltages associated with the N electrodes may be referred to as $V_1$, $V_2$, ..., and $V_N$. The voltage $V_1$ may refer to the voltage at the first electrode. The voltage $V_2$ may refer to the voltage at the second electrode. Similarly, the voltage $V_N$ may refer to the voltage at the Nth electrode.

The plurality of electrodes may be arranged along a lead. For example, the electrodes may be arranged in a line along the lead. In one example, the lead may be a linear lead and the electrodes may be cylindrical electrodes arranged in a line along the lead. Since the electrodes may be arranged in a line along the lead, two of the electrodes may be described as end electrodes. Each of the end electrodes may be adjacent to only a single electrode. The electrodes between the end electrodes may each be arranged between two adjacent electrodes.

As described herein, the processing module may determine a voltage difference between each of the adjacent electrodes on the lead. In examples where the lead includes N electrodes, the processing module may determine N-1 voltage differences. For example, the processing module may determine voltage differences $V_1$-$V_2$, $V_2$-$V_3$, $V_3$-$V_4$,..., and $V_{N-1}$-$V_N$. In a more specific example, where the lead includes four electrodes arranged along the lead, the processing module may determine three voltage differences along the lead. For example, the processing module may determine voltage differences $V_1$-$V_2$, $V_2$-$V_3$, and $V_3$-$V_4$.

The processing module may determine CSD values based on the voltage differences between the adjacent electrodes. In some examples, the CSD values may be the second voltage difference along the electrodes. Each of the second voltage differences may be a difference between the voltage differences. In other words, in some examples, the CSD values may be the differences between the voltage differences along the lead. In a more specific example, the two CSD values for a four electrode system would be $(V_1$-$V_2)$-$(V_2$-$V_3)$ and $(V_2$-$V_3)$-$(V_3$-$V_4)$.

The processing module may determine a CSD value for each electrode that is between two other electrodes. In general, in systems that include N electrodes, the processing module may determine N-2 CSD values, each of which may be associated with a different one of the electrodes. The end electrodes, i.e., the electrodes not arranged between two adjacent electrodes, may not have associated CSD values in some examples because the outside electrodes may not be associated with two different voltage difference values.

As described above, each CSD value may indicate the relative strength of a current source or current sink in the proximity of the electrode associated with the CSD value. For example, a positive CSD value associated with an electrode may indicate the presence of a current source in proximity to the electrode associated with the positive CSD value. As another example, a negative CSD value associated with an electrode may indicate the presence of a current sink in proximity to the electrode. The magnitude of the CSD value may indicate the relative strength of the current source or sink in the proximity of the electrode.

The processing module may control the electrical stimulation delivered to the patient via the electrodes based on the CSD values associated with the electrodes. For example, the stimulation device may determine which electrode or electrodes of the N electrodes to use for delivery of electrical stimulation based on the CSD values. The stronger sources and sinks, as indicated by larger CSD values, may be the origin of some pathological activity contributing to the patient's condition. Accordingly, delivering the stimulation via the electrodes associated with the larger CSD values may tend to modify some of the pathological activity and alleviate some of the patient's condition.

In examples in which the processing module is configured to control delivery of electrical stimulation using a single electrode of the N electrodes on the lead (e.g., during monopolar stimulation), the processing module may select the electrode having the largest CSD value, by magnitude, and control the delivery of electrical stimulation via that electrode while controlling the stimulation generator to refrain from delivering electrical stimulation using other electrodes. In some examples, the processing module may select an electrode having the largest positive CSD value (e.g., the largest source) and control delivery of electrical stimulation to that electrode while controlling the stimulation generator to refrain from stimulating using the other electrodes. In some examples, the processing module may select an electrode having the largest negative CSD value (e.g., the largest sink) and control delivery of electrical stimulation to that electrode while controlling the stimulation generator to refrain from stimulating using the other electrodes.

In examples in which the stimulation device is configured to deliver stimulation using two electrodes along a lead (e.g., in bipolar stimulation), the processing module may select the two electrodes of the N electrodes based on the CSD values associated with the two electrodes. For example, the processing module may select the two electrodes having the largest CSD values, by magnitude, and control the delivery of electrical stimulation via the two electrodes while controlling the stimulation generator to refrain from delivering electrical stimulation using electrodes other than the two selected electrodes. In other examples, the processing module may select the two electrodes having the largest positive CSD values (e.g., the largest sources) and control delivery of electrical stimulation to the two electrodes while controlling the stimulation generator to refrain from stimulating other electrodes. In other examples, the processing module may select the two electrodes having the largest negative CSD values (e.g., the largest sinks) and control delivery of electrical stimulation to the two electrodes while controlling the stimulation generator to refrain from stimulating via other electrodes. In still other examples, the processing module may select the largest positive CSD value and the largest negative CSD value and control delivery of electrical stimulation to the electrodes having the largest positive and negative CSD values while controlling the stimulation generator to refrain from stimulating via other electrodes.

In some examples, the stimulation device may not select some electrodes for delivery of stimulation while controlling the stimulation generator to refrain from stimulating those electrodes which are not selected. Instead, the stimulation device may apportion the electrical stimulation to the electrodes based on the CSD values associated with the electrodes. For example, the processing module may control the stimulation generator to apportion a relatively greater amount of electrical stimulation current to electrodes having relatively greater CSD values and apportion a relatively lesser amount of stimulation current to electrodes having smaller CSD values.

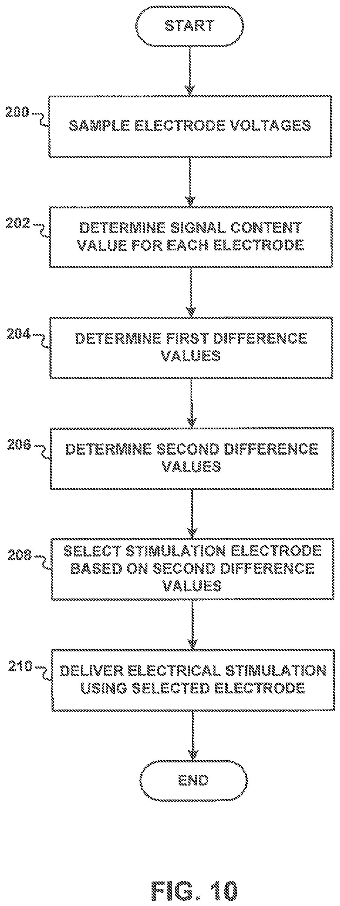

In some examples, using second difference values for selection of stimulation electrodes may be extended to second difference values other than second voltage differences. For example, as described herein with respect to FIG. 10, the processing module may select stimulation electrodes based on second differences of other parameters associated with electrodes. In one example, the processing module may determine an amount of signal content (e.g., power) associated with each electrode within a frequency band and select electrodes for stimulation based on the second differences of those values.

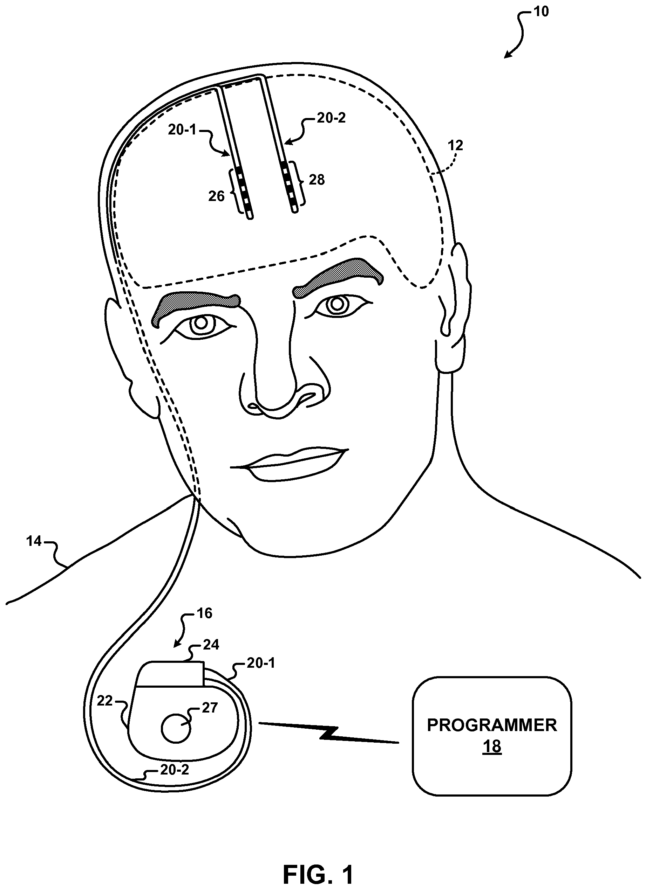

FIG. 1 is a conceptual diagram illustrating an example therapy system 10 that is implanted proximate to brain 12 of patient 14 in order to help manage a patient condition, such as pain, psychiatric disorder, movement disorder, or seizure disorder. Therapy system 10 includes implantable medical device (IMD) 16 for delivering electrical stimulation, a medical device programmer 18, and leads 20-1, 20-2 (collectively "leads 20").

IMD 16 includes a housing 22 configured for implantation within patient 14. Although IMD 16 is configured for implantation within patient 14, in other examples, the techniques of the present disclosure may be implemented in an electrical stimulation device that is external to patient 14. Connector block 24 is coupled to housing 22. Connector block 24 is configured to receive leads 20. Leads 20 are coupled to IMD 16 via connector block 24, e.g., using set screws. Leads 20 each include conductors that extend along the length of leads 20 and terminate at electrodes 26-1, 26-2, 26-3, 26-4 (collectively "electrodes 26) and 28-1, 28-2, 28-3, 28-4 (collectively "electrodes 28"). Connector block 24 may include electrical contacts configured to contact conductors of leads 20. The electrical contacts of connector block 24 may electrically couple electrodes 26, 28 to electronics of IMD 16. In some examples, IMD 16 may include a housing electrode 27.

Although two leads 20-1, 20-2 are illustrated in FIG. 1, techniques of the present disclosure may be applicable to IMDs having more or fewer than two leads. Although IMD 16 is illustrated as delivering electrical stimulation and/or sensing electrical physiological signals using four electrodes per lead, the techniques of the present disclosure may be applicable to IMDs having more or fewer than four electrodes per lead. For example, the techniques of the present disclosure may be applicable to IMDs that deliver stimulation and/or sense via 16-32 electrodes, or more.

IMD 16 includes a stimulation generator 30 that delivers electrical stimulation to one or more regions of brain 12 via electrodes 26, 28. In the example shown in FIG. 1, therapy system 10 may be referred to a deep brain stimulation (DBS) system because IMD 16 provides electrical stimulation therapy to tissue within brain 12, e.g., a tissue site under the dura mater of brain 12. Leads 20 may be positioned to deliver electrical stimulation to one or more target tissue sites within brain 12 to manage patient symptoms associated with a patient disorder. In the example shown in FIG. 1, leads 20 are implanted within the right and left hemispheres, respectively, of brain 12 in order to deliver electrical stimulation to one or more regions of brain 12, which may be selected based on many factors, such as the type of patient condition for which therapy system 10 is implemented to manage. Leads could also be implanted within a same hemisphere of the brain in other examples.

Although a DBS system is illustrated in the disclosure, it is contemplated that the techniques of the present disclosure may be implemented in other types of electrical stimulation applications used to treat various types of patient conditions. For example, the techniques of the present disclosure may be implemented in spinal cord stimulation systems, gastric stimulation systems, peripheral nerve stimulation, peripheral nerve field stimulation, or systems that electrically stimulate any other suitable nerve, organ, muscle, or muscle group to treat a condition of patient 14. Although therapy system 10 may be used to treat conditions such as movement disorders or other neurological disorders, it is contemplated that the techniques of the present disclosure may be implemented in devices used to treat other types of patient conditions, such as pain, urinary or fecal incontinence, or obesity, as examples.

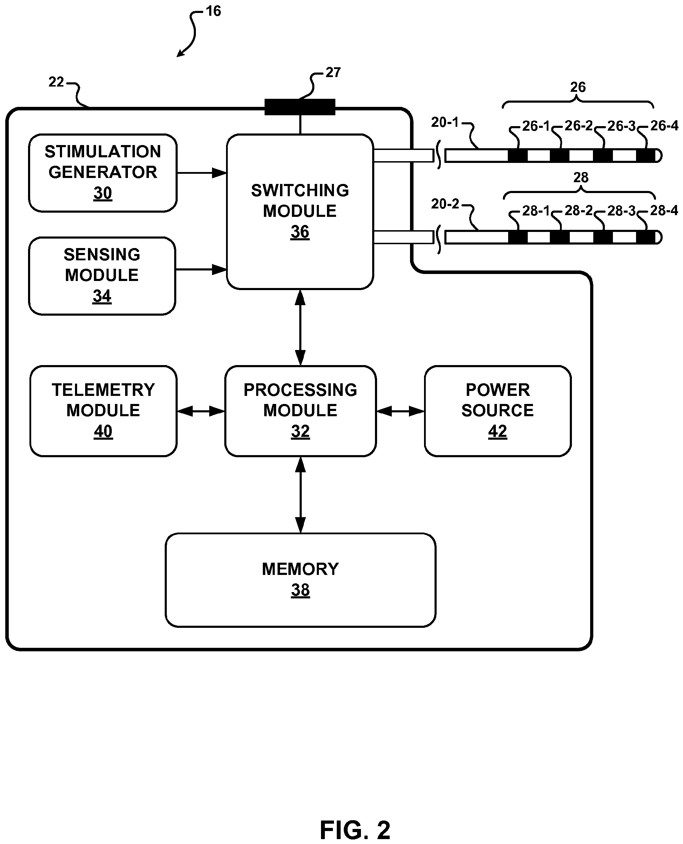

IMD 16 generates the electrical stimulation according to one or more therapy parameters, which may be arranged in a therapy program (or a parameter set) stored in memory 38 (FIG. 2) of IMD 16. IMD 16 may deliver electrical stimulation according to a variety of different parameters, such as voltage or current pulse amplitude, pulse rate, pulse width, burst rate, burst size, and a continuous waveform shape. Electrical stimulation parameters may define a variety of different waveforms, such as rectangular waveforms, sinusoidal waveforms, or ramped signals, as examples. In addition, the electrical stimulation parameters may define different electrode combinations, which can include selected electrodes and their respective polarities. As described herein, processing module 32 (FIG. 2) may set the electrode combinations based on CSD values associated with electrodes 26, 28.

IMD 16 includes a sensing module 34 (FIG. 2) that may sense electrical physiological signals (e.g., bioelectrical brain signals) of patient 14 using electrodes 26, 28. In therapy system 10, electrodes 26, 28 placed near neurons may sense extracellular neuronal potentials that are indicative of neuronal function. The extracellular potentials generated in patient 14 may set up a plurality of current sources and sinks within the extracellular environment. When implanted in the brain as illustrated in FIG. 1, the current sources/sinks may be generated by groups of neurons in the brain. The CSD values may indicate the location and magnitude of the current sources and sinks in proximity to the electrodes 26, 28. For example, a positive CSD value may indicate that a current source is in proximity to an electrode associated with the CSD value, while a negative value may indicate that a current sink is in proximity to the electrode associated with the CSD value. The magnitude of a CSD value may indicate the strength of the source/sink.

In the example shown in FIG. 1, electrodes 26, 28 may be positioned to sense bioelectrical brain signals within brain 12. In some examples, the bioelectrical signals sensed within brain 12 may reflect changes in electrical current produced by the sum of electrical potential differences across brain tissue. Examples of bioelectrical brain signals include, but are not limited to, electrical signals generated from local field potentials (LFP) sensed within one or more regions of brain 12, such as an electroencephalogram (EEG) signal, or an electrocorticogram (ECoG) signal.

Although the IMD 16 is illustrated as sensing bioelectrical brain signals, in other examples, the arrangement of electrodes disclosed herein can be used to sense other electrical physiological signals, such as electrocardiogram (ECG) signals, electrogram (EGM) signals, electromyogram (EGM) signals (e.g., intrinsic muscular depolarizations), and the like. Although illustrated as internal, electrodes 26, 28 may also be positioned to sense an electrical physiological signal in other locations within patient 12 or external to patient 12. Accordingly, the techniques for selecting stimulation electrodes based on CSD analysis may be implemented in other stimulation applications.

As described herein, each of electrodes 26, 28 may be used to deliver electrical stimulation to patient 14. Additionally, each of electrodes 26, 28 may be used to sense electrical physiological signals. Although each of electrodes 26, 28 may be used to deliver electrical stimulation and sense electrical physiological activity, in other examples, some of electrodes 26, 28 may be dedicated to stimulation while others are dedicated to sensing electrical physiological signals. IMD 16 may include a switching module 36 that may be configured to connect either stimulation generator 30 or sensing module 34 to electrodes 26, 28 in order to transition electrodes 26, 28 between sensing and stimulation.

External programmer 18 wirelessly communicates with IMD 16 to provide or retrieve therapy information. Programmer 18 is an external computing device that the user, e.g., the clinician and/or patient 14, may use to communicate with IMD 16. For example, programmer 18 may be a clinician programmer that the clinician uses to communicate with IMD 16 and program one or more therapy programs for IMD 16. Alternatively, programmer 18 may be a patient programmer that allows patient 14 to select programs and/or view and modify therapy parameters.

FIG. 2 is a functional block diagram illustrating components of example IMD 16. In the example shown in FIG. 2, IMD 16 includes a processing module 32, memory 38, stimulation generator 30, sensing module 34, switching module 36, telemetry module 40, and power source 42. In some examples, IMD 16 may include a sensor (not shown), such as a motion sensor (e.g., an accelerometer), that generates a signal indicative of patient activity (e.g., patient movement or patient posture transitions).

Processing module 32, stimulation generator 30, sensing module 34, switching module 36, memory 38, and telemetry module 40 represent functionality that may be included in IMD 16 of the present disclosure. Processing module 32, stimulation generator 30, sensing module 34, switching module 36, memory 38, and telemetry module 40 of the present disclosure may include any discrete and/or integrated electronic circuit components that implement analog and/or digital circuits capable of producing the functions described herein. For example, processing module 32, stimulation generator 30, sensing module 34, switching module 36, memory 38, and telemetry module 40 may include analog circuits, e.g., amplification circuits, filtering circuits, and/or other signal conditioning circuits. Processing module 32, stimulation generator 30, sensing module 34, switching module 36, memory 34, and telemetry module 38 may also include digital circuits, e.g., combinational or sequential logic circuits, memory, or the like. Memory may include any volatile, non-volatile, magnetic, or electrical media, such as a random access memory (RAM), read-only memory (ROM), non-volatile RAM (NVRAM), electrically-erasable programmable ROM (EEPROM), Flash memory, or any other memory device. Furthermore, memory may include instructions that, when executed by one or more processing circuits, cause processing module 32, stimulation generator 30, sensing module 34, switching module 36, memory 34, and telemetry module 38 to perform various functions described herein.

The functions attributed to processing module 32, stimulation generator 30, sensing module 34, switching module 36, memory 34, and telemetry module 38 herein may be embodied as one or more processors, hardware, firmware, software, or any combination thereof. Depiction of different features as separate components is intended to highlight different functional aspects and does not necessarily imply that such components must be realized by separate hardware or software components. Rather, functionality associated with one or more components may be performed by separate hardware or software components, or integrated within common or separate hardware or software components.

Memory 38 may store therapy programs. A therapy program may define electrical stimulation parameters, such as electrode polarity, current or voltage amplitude, and, if stimulation generator 30 generates and delivers stimulation pulses, the therapy programs may define values for a pulse width, pulse rate, burst length or width, and duty cycle of a stimulation signal. In some examples, the therapy programs may be stored as a therapy group, which defines a set of therapy programs with which stimulation may be generated. The stimulation signals defined by the therapy programs of the therapy group may be delivered together on an overlapping or non-overlapping (e.g., time-interleaved) basis. Therapy programs may also define electrode combinations used to deliver electrical stimulation to patient 14. As described herein, processing module 32 may set the electrode combinations based on CSD values associated with electrodes 26, 28.

IMD 16 is coupled to leads 20, which include electrodes 26, 28. Electrodes 26, 28 may be electrically coupled to switching module 36 via conductors within the respective leads 20. Switching module 36 may be a switch array, switch matrix, multiplexer, or any other type of switching circuit configured to couple stimulation generator 30 and/or sensing module 34 to selected electrodes 26, 28. Each of electrodes 26, 28 may be coupled to separate conductors so that electrodes 26, 28 may be individually selected, or in some examples, two or more electrodes 26 and/or two or more electrodes 28 may be coupled to a common conductor. Electrodes may be evenly spaced apart in some examples such that the distance between each of electrodes 26, 28 is equal along leads 20-1, 20-2. In some examples, electrodes 26, 28 may be ring electrodes that extend around the circumference of the lead body. In other examples, some or all of electrodes 26, 28 may be segmented electrodes that only extend part-way around the circumference of a lead. For instance, three or four segmented electrodes may each be located at a same axial position along the length of the lead but at different angular positions around the circumference of the lead.

Processing module 32 may control switching module 36 in order to select which combination of electrodes are used during electrical stimulation and electrical sensing. Processing module 32 may control switching module 36 to connect any combination of electrodes 26, 28 to stimulation generator 30 or sensing module 34. In some examples, processing module 32 may control switching module 36 to connect all of electrodes 26, 28 to stimulation generator 30 at one time. In some examples, processing module 32 may control switching module 36 to connect all of electrodes 26, 28 to sensing module 34 at another time. In other examples, processing module 32 may control switching module 36 to connect some of electrodes 26, 28 to stimulation generator 30 and the remaining ones of electrodes 26, 28 to sensing module 34 at one time.

To deliver electrical stimulation to patient 14, processing module 32 may configure switching module 36 to connect stimulation generator 30 to electrodes 26, 28. Stimulation generator 30 is coupled to electrodes 26, 28 via switching module 36 and conductors within leads 20. Processing module 32 controls stimulation generator 30 to generate and deliver electrical stimulation signals to patient 14 according to selected therapy parameters when stimulation generator 30 is coupled to electrodes 26, 28. For example, processing module 32 may control stimulation generator 30 according to therapy programs stored in memory 38 to apply particular stimulation parameter values such as current or voltage amplitude. Each stored therapy program may define a particular set of electrical stimulation parameter values, such as a stimulation electrode combination, current or voltage amplitude, frequency (e.g., pulse rate in the case of stimulation pulses), and pulse width. In some examples, individual therapy programs may be stored as a therapy group, which defines a set of therapy programs with which stimulation may be generated. Stimulation generator 30 may be capable of delivering a single stimulation pulse, multiple stimulation pulses, or a continuous signal at a given time via a single electrode combination.

Processing module 32 may configure switching module 36 to connect sensing module 34 to electrodes 26, 28. Sensing module 34 is coupled to electrodes 26, 28 via switching module 36 and conductors within leads 20. Sensing module 34 may include circuitry that senses the electrical physiological activity of a particular region, e.g., motor cortex, within brain 12 via electrodes 26, 28. Sensing module 34 may acquire (e.g., sample) the electrical physiological signals of patient 12 at a sampling rate, which may be adjustable. Sensing module 34 may include circuitry for determining a voltage difference between two electrodes (a bipolar configuration) or between at least one electrode of leads 20 and a reference electrode separate from leads 20, such as housing electrode 27 (a monopolar configuration). In a bipolar configuration, one of electrodes 26, 28 may act as a reference electrode, and, in a monopolar configuration, housing electrode 27 may act as a reference electrode. The sampled (i.e., digitized) physiological electrical signals may be referred to herein as sensing data. The sensing data may be digital data that indicates voltages associated with electrodes 26, 28. Processing module 32 may receive the sensing data from sensing module 34. Processing module 32 may apply additional digital signal processing (e.g., filtering) to the sensing data received from sensing module 34 in some examples.

Telemetry module 40 supports wireless communication between IMD 16 and programmer 18 or another computing device. Processing module 32 of IMD 16 may receive, as updates to programs, values for various stimulation parameters such as amplitude and electrode combination, from programmer 18 via telemetry module 38. The updates to the therapy programs may be stored within memory 38.

Power source 42 delivers operating power to various components of IMD 16. Power source 42 may include a small rechargeable or non-rechargeable battery and a power generation circuit to produce the operating power. In some examples, stimulation generator 30 may include a circuit that boosts the voltage output by power source 42. The boosted voltage may be used by stimulation generator 30 to generate current and/or voltage delivered to patient 14. In examples where stimulation generator 30 includes a circuit that boosts voltage, processing module 32 may control the level of voltage provided by the circuit.

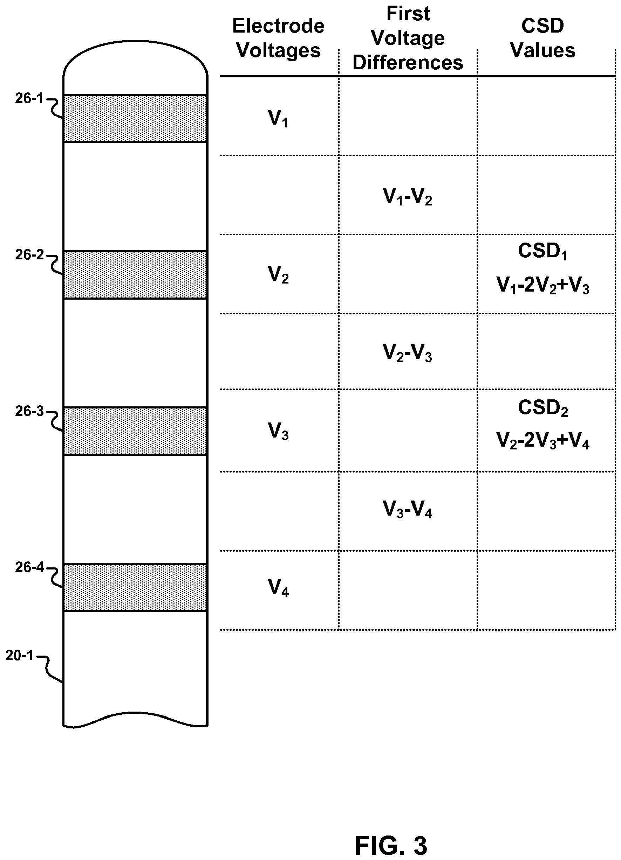

Processing module 32 may receive the sensing data from sensing module 34 and determine CSD values based on the sensing data. Processing module 32 may then control stimulation generator 30 and switching module 36 to deliver electrical stimulation to patient 14 based on the CSD values. Determination of CSD values and the control of electrical stimulation based on the determined CSD values is described with reference to FIGS. 3-7.

An example determination of CSD values is now described with reference to FIG. 2 and FIG. 3. To determine CSD values associated with electrodes 26, 28, processing module 32 may initially determine voltages associated with electrodes 26, 28. Processing module 32 may then determine voltage differences between adjacent ones of electrodes 26, 28. For example, processing module 32 may determine voltage differences between each pair of adjacent electrodes along leads 20-1, 20-2.

Processing module 32 and sensing module 34 may be configured to determine voltages associated with electrodes 26, 28 in a variety of different ways. Initially, processing module 32 may configure switching module 36 to connect sensing module 34 to electrodes 26, 28 in order to measure voltages associated with electrodes 26, 28.

Sensing module 34 may be configured to measure voltages associated with electrodes 26, 28 in a variety of different ways. In general, sensing module 34 may sample the voltages at electrodes 26, 28 in any manner that allows processing module 32 to determine the voltage difference between adjacent ones of electrodes 26, 28. In some examples, sensing module 34 may measure the voltages associated with electrodes 26, 28 relative to a single sensing electrode, which may be selected from any of electrodes 26, 28, 27. In these examples, the voltages associated with electrodes 26, 28 may be voltages of electrodes 26, 28 with respect to the reference electrode. In one example, processing module 32 may measure voltages associated with electrodes 26, 28 relative to housing electrode 27. However, measurement of electrode voltages relative to housing electrode 27 may be prone to picking up electrical noise.

In other examples, sensing module 34 may measure voltages associated with electrodes 26, 28 with respect to a reference electrode chosen from electrodes 26, 28. For example, sensing module 34 may measure the voltages of each of electrodes 26-1, 26-2, 26-3 relative to electrode 26-4. In other examples, sensing module 34 may be connected to electrodes 26, 28 such that sensing module 34 directly measures voltage differences between adjacent electrodes. For example, sensing module 34 may connect to electrodes 26, 28 such that sensing module 34 directly measures the voltage differences $V_1$-$V_2$, $V_2$-$V_3$, and $V_3$-$V_4$ (FIG. 3).

Sensing module 34 may send sensing data to processing module 32 that indicates the voltages associated with electrodes 26, 28. In some examples, sensing data may include voltages associated with electrodes 26, 28 with respect to a single reference electrode, e.g., housing electrode 27, or one of electrodes 26, 28. In other examples, sensing data may include the voltage differences between adjacent electrodes.

In some examples, sensing module 34 may sample voltages associated with electrodes 26, 28 at approximately the same time. For example, sensing module 34 may sample voltages for each of electrodes 26, 28 in parallel, e.g.,

using multiple analog-to-digital (A/D) converters. In other examples, sensing module 34 may sequentially sample voltages one after another, e.g., using a single A/D converter. Regardless of the sampling technique used, sensing module 34 may sample voltages for each of electrodes 26, 28 such that sensing module 34 may provide sensing data that processing module 32 may use to determine the voltage differences between adjacent electrodes.

FIG. 3 shows example electrode voltages and voltage differences for electrodes 26 of lead 20-1. Each of electrodes 26 of lead 20-1 may be associated with voltages $V_1$-$V_4$. In some examples, as described above, sensing module 34 may sample each of voltages $V_1$-$V_4$ relative to a reference node, such as housing electrode 27, or any of electrodes 26, 28. The sampled voltages $V_1$-$V_4$, sampled with respect to a reference electrode, may be thought of as a voltage profile along lead. In other examples sensing module 34 may directly sample the voltage differences between adjacent electrodes to directly sample voltage differences $V_1$-$V_2$, $V_2$-$V_3$, $V_3$-$V_4$. The voltage differences $V_1$-$V_2$, $V_2$-$V_3$, $V_3$-$V_4$ between each of electrodes 26 may be referred to herein as "first voltage differences."

Sensing module 34 may generate sensing data based on the sampled voltages. Processing module 32 may determine the first voltage differences based on the sensing data. In examples where sensing data includes electrode voltages $V_1$-$V_4$ with respect to a reference electrode, processing module 32 may determine the first voltage differences based on the electrode voltages $V_1$-$V_4$. In other examples, in which sensing data includes the first voltage differences, processing module 32 may directly determine the first voltage differences from the sensing data without further processing of the sensing data.

Processing module 32 may then determine CSD values based on the first voltage differences. In some examples, the CSD values may be second voltage differences. In these examples, processing module 32 may determine CSD values by subtracting first voltage differences from one another. For example, as illustrated in FIG. 3, processing module 32 may subtract first voltage difference ($V_2$-$V_3$) from first voltage difference ($V_1$-$V_2$) to determine a first CSD value ($V_1$-$2V_2$+$V_3$) associated with electrode 26-2. In a similar manner, processing module 32 may subtract first voltage difference ($V_3$-$V_4$) from first voltage difference ($V_2$-$V_3$) to determine a second CSD value ($V_2$-$2V_3$+$V_4$) associated with electrode 26-3.

A CSD value may indicate the location and relative magnitude of current sources and current sinks in the tissue surrounding the electrodes 26. For example, the sign (e.g., positive or negative) of $CSD_1$ may indicate whether a source or sink is in the vicinity of electrode 26-2. Similarly, the sign of $CSD_2$ may indicate whether a source or sink is in the vicinity of electrode 26-3. Additionally, the relative magnitudes of CSD values $CSD_1$ and $CSD_2$ may indicate the size of the current sources/sinks in the vicinity of $CSD_1$ and $CSD_2$. For example, if both $CSD_1$ and $CSD_2$ have positive signs, the larger CSD value may indicate the larger current source.

In some examples, sensing module 34 and/or processing module 32 may filter sensed electrical physiological signals. For example, sensing module 34 and/or processing module 32 may filter sensed physiological signals in order to process selected frequency content that may be associated with some pathological activity contributing to the patient's condition. In some examples, sensing module 34 may include one or more filter circuits that filter electrical signals acquired via electrodes 26, 28. In some examples, processing module 32 may implement digital filters that filter sensing data received from sensing module 34.

In some examples, sensing module 34 and/or processing module 32 may implement a bandpass filter function (e.g., selectable by a user) to focus on a frequency band of interest. For example, sensing module 34 and/or processing module 32 may implement a bandpass filter function that attenuates acquired electrical physiological signals outside of the beta frequency band (12-30 Hz) in order to focus processing on signal content that may be associated with the beta frequency band (e.g., Parkinson's disease). Although sensing module 34 and/or processing module 32 may implement a bandpass filter function, it is contemplated that sensing module 34 and/or processing module 32 may implement a variety of different filter functions, such as low pass filters, high pass filters, and bandstop filters, for example.

In some examples, processing module 32 may determine CSD values based on the filtered electrical physiological signals to detect current sources/sinks associated with certain pathological activity. For example, processing module 32 may determine CSD values based on electrical physiological signals within the beta frequency band. In this example, the determined CSD values within the beta frequency band may indicate the relative strength of current sources/sinks associated with pathological activity within the beta frequency band (e.g., Parkinson's disease). Processing module 32 may select the electrodes for delivery of electrical stimulation based on the CSD values associated with the selected frequency band. Delivering electrical stimulation via the electrodes associated with the larger CSD values within the frequency band may tend to modify some of the pathological activity and alleviate some of the patient's condition associated with Parkinson's disease.

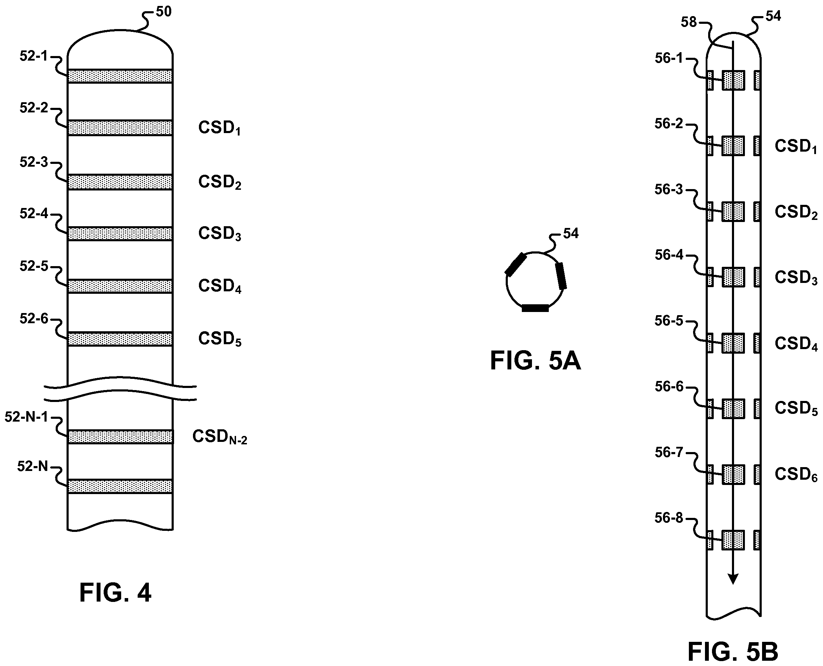

IMD 16 may be configured to determine CSD values for lead and electrode configurations other than those illustrated in FIGS. 1-3. FIG. 4 shows a more generalized example of a linear lead having more cylindrical electrodes than those shown in FIGS. 1-3. Lead 50 of FIG. 4 includes cylindrical electrodes 52-1, 52-2, 52-N (collectively "electrodes 52"). In examples where lead 50 is coupled to IMD 16, processing module 32 may determine N voltages corresponding to electrodes 52. Processing module 32 may determine N-1 first voltage differences and N-2 CSD values. The N-2 CSD values are labeled as $CSD_1$, $CSD_2$,..., and $CSD_{N-2}$. Each of the CSD values of FIG. 4 may correspond to one of electrodes 52-2 to 52-N-1. Accordingly, the techniques of the present disclosure for determining CSD values may be extended to linear leads having any number of cylindrical electrodes. In some examples, IMD 16 may be configured to receive a single lead including N cylindrical electrodes and determine CSD values for N-2 of the N cylindrical electrodes. In other examples, IMD 16 may be configured to receive two or more leads similar to lead 50 illustrated in FIG. 4 and determine N-2 CSD values for each of the two or more leads.

FIG. 5A and FIG. 5B show top and side views, respectively, of another lead 54 that includes different types of electrodes than the cylindrical electrodes illustrated in FIGS. 1-4. The electrodes of FIGS. 5A-5B may be referred to as segmented electrodes, which are described above. Lead 54 includes three columns of the segmented electrodes.

Processing module 32 may determine CSD values in a similar manner as described with respect to FIGS. 3-4. With respect to FIG. 5B, processing module 32 may determine CSD values for some of electrodes 56-1, 56-2,..., 56-8 (collectively "electrodes 56") along the line indicated at 58. As described above, sensing module 34 may sample voltages associated with electrodes 56 and generate sensing data based on the sampled voltages. In the example of FIG. 5B, sensing module 34 may sample voltages for each of electrodes 58. Processing module 32 may determine the first voltage differences based on the sensing data and then determine six CSD values (e.g., second voltage differences) along lead 54 based on the first voltage differences. The six CSD values $CSD_1$-$CSD_6$ of FIG. 5B may be associated with electrodes 56-2, 56-3,..., and 56-7, respectively. Although determination of CSD values for a single column of electrodes is illustrated and described with respect to FIG. 5B, processing module 32 may determine CSD values for each of the columns of electrodes on lead 54.

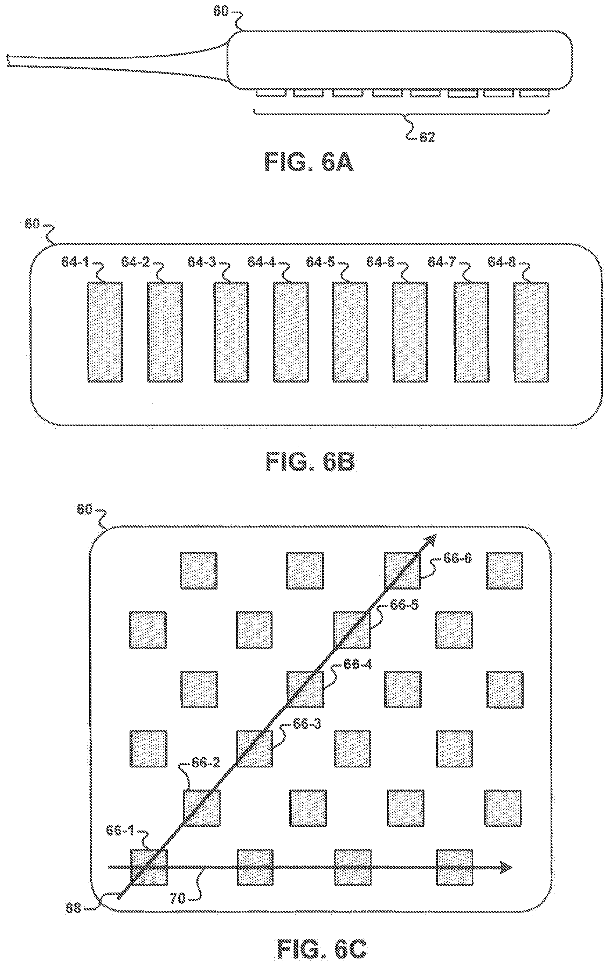

FIGS. 6A-6C illustrate electrodes arranged along a paddle lead 60. FIG. 6A shows a side view of an example paddle lead 60 that includes electrodes 62 on one surface, e.g., a bottom surface, of paddle lead 62. FIG. 6B shows an example arrangement of electrodes 64-1, 64-2,..., 64-8 (collectively "electrodes 64") on paddle lead 60. FIG. 6C shows another example arrangement of electrodes 66-1, 66-2,..., 66-N (collectively, "electrodes 66") on paddle lead 60. The arrangement of electrodes illustrated in each of FIGS. 6A-6C may be referred to as an "electrode array." The arrangement of electrodes illustrated in FIG. 6C may be referred to as a two-dimensional electrode array.

Processing module 32 may determine CSD values for electrodes 64 in FIG. 6B in a similar manner as described with respect to FIGS. 3-4. With respect to FIG. 6B, processing module 32 may determine CSD values for some of electrodes 64. As described above, sensing module 34 may sample voltages associated with electrodes 64 and generate sensing data based on the sampled voltages. In the example of FIG. 6B, sensing module 34 may sample voltages for each of electrodes 64. Processing module 32 may determine the first voltage differences based on the sensing data and then determine six CSD values (e.g., second voltage differences) based on the first voltage differences. The six CSD values for FIG. 6B may be associated with electrodes 64-2, 64-3,..., and 64-7.

In the example illustrated by FIG. 6C, processing module 32 may determine CSD values for electrodes along a variety of different paths. In one example, processing module 32 may determine CSD values for electrodes 66-2, 66-3, 66-4, and 66-5 along line 68 in FIG. 6C in a similar manner as described with respect to FIGS. 3-4. As described above, sensing module 34 may sample voltages associated with electrodes 66 and generate sensing data based on the sampled voltages. Processing module 32 may then determine the first voltage differences based on the sensing data and then determine four CSD values (e.g., second voltage differences) based on the first voltage differences. The four CSD values for FIG. 6C may be associated with electrodes 66-2, 66-3, 66-4, and 66-5.

In other examples, processing module 32 may determine CSD values along other lines of electrodes included on paddle lead 60 of FIG. 6C. For example, processing module 32 may determine two CSD values for the four electrodes arranged along line 70. Although processing module 32 may determine CSD values for electrodes arranged along lines 68, 70, in some examples, processing module 32 may determine CSD values along other lines not illustrated in FIG. 6C.

Accordingly, processing module 32 may determine CSD values for a variety of different types and arrangements of electrodes. After determining CSD values for electrodes, processing module 32 may control stimulation generator 30 to deliver electrical stimulation via electrodes based on the CSD values associated with the electrodes. Control of electrical stimulation by processing module 32 based on the CSD values associated with electrodes is now described with reference to lead 50 of FIG. 4.

In examples in which processing module 32 is configured to control delivery of electrical stimulation using a single electrode of electrodes 52 on lead 50, e.g., during monopolar stimulation while using housing electrode 27, processing module 32 may select the electrode having the largest CSD value, by magnitude, for the delivery of stimulation. After selecting a stimulation electrode based on the CSD value associated with the electrode, processing module 32 may control stimulation generator 30 to deliver electrical stimulation via the selected electrode. Processing module 32 may also control stimulation generator 30 to refrain from delivering electrical stimulation using electrodes other than the selected electrode. In one specific example, with respect to FIG. 4, if processing module 32 is configured to deliver electrical stimulation using a single electrode of electrodes 52 in a monopolar configuration, and processing module 32 determines that $CSD_4$ is the largest CSD value of the N-2 CSD values, then processing module 32 may control stimulation generator 30 to deliver stimulation to patient 14 via electrode 52-5. While controlling delivery of stimulation to electrode 52-5, which is associated with the largest CSD value, processing module 32 may also control stimulation generator 30 to refrain from stimulating electrodes other than electrode 52-5.

In some examples, instead of selecting the largest CSD value by magnitude, processing module 32 may select an electrode having the largest positive CSD value (e.g., the largest source) and control delivery of electrical stimulation to that electrode while controlling stimulation generator 30 to refrain from delivering stimulation to other electrodes. In some examples, processing module 32 may select an electrode having the largest negative CSD value (e.g., the largest sink) and control delivery of electrical stimulation to that electrode while controlling stimulation generator 30 to refrain from delivering stimulation to other electrodes.

In examples in which processing module 32 is configured to control delivery of electrical stimulation using two electrodes, e.g., as in bipolar stimulation, processing module 32 may select the two electrodes of electrodes 52 based on the CSD values associated with the two electrodes. For example, processing module 32 may select the two electrodes having the largest CSD values, by magnitude, and control the delivery of electrical stimulation via the two electrodes while controlling stimulation generator 30 to refrain from delivering electrical stimulation using electrodes other than the two selected electrodes. In other examples, processing module 32 may select the two electrodes having the largest positive CSD values (e.g., the largest sources) and control delivery of electrical stimulation to the two electrodes while controlling stimulation generator 30 to refrain from delivering electrical stimulation to other electrodes. In other examples, processing module 32 may select the two electrodes having the largest negative CSD values (e.g., the largest sinks) and control delivery of electrical stimulation to the two electrodes while controlling stimulation generator 30 to refrain from delivering electrical stimulation to other electrodes.

In some examples, processing module 32 may not select some electrodes for delivery of stimulation while inhibiting stimulation to those electrodes which are not selected. Instead, processing module 32 may control stimulation generator 30 to apportion the electrical stimulation to electrodes 52 based on the CSD values associated with electrodes 52. For example, processing module 32 may control stimulation generator 30 to apportion a relatively greater amount of electrical stimulation to electrodes having relatively greater CSD values and apportion a relatively lesser amount of stimulation to electrodes having smaller CSD values.

In some examples, processing module 32 may select stimulation electrodes from more than one lead. For example, when processing module 32 is configured to control delivery of electrical stimulation using electrodes from two different leads such as leads 20, processing module 32 may select a stimulation electrode from lead 20-1 based on CSD values associated with electrodes 26 on lead 20-1 and select a stimulation electrode from lead 20-2 based on CSD values associated with electrodes 28 on lead 20-2. Although delivery of electrical stimulation based on CSD values is described above with respect to leads 20, 50 of FIGS. 1-4, processing module 32 may control the delivery of electrical stimulation to electrodes of leads 58, 60 in a similar manner.

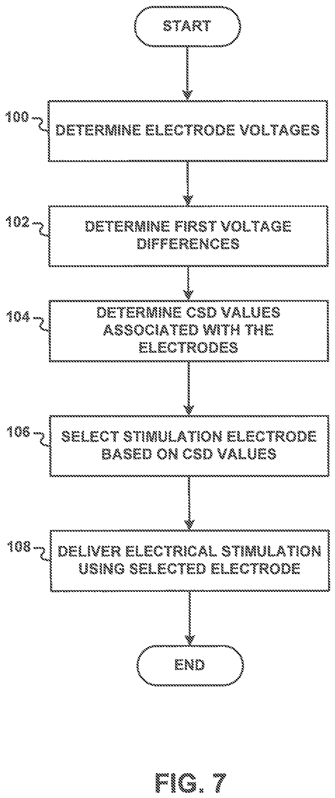

FIG. 7 shows a method for selecting stimulation electrodes based on CSD values associated with the electrodes. The method of FIG. 7 is described with reference to IMD 16 of FIG. 2 and lead 20-1 of FIG. 3. For purposes of describing the method of FIG. 7, it may be assumed that IMD 16 is configured to deliver monopolar electrical stimulation using a single one of electrodes 26 in combination with housing electrode 27.

Processing module 32 may initially determine voltages associated with electrodes 26 based on sensing data received from sensing module 34 (100). Processing module 32 may then determine first voltage differences between adjacent electrodes of electrodes 26 (102). For example, processing module 32 may determine first voltage differences $V_1$-$V_2$, $V_2$-$V_3$, $V_3$-$V_4$.

Processing module 32 may then determine CSD values associated with electrodes 26-2, 26-3 (104). In some examples, the CSD values may be second voltage differences of the voltages along electrodes 26. In these examples, processing module 32 may determine the CSD values by subtracting one first voltage difference from another first voltage difference. For example, as illustrated in FIG. 3, processing module 32 may subtract the first voltage difference ($V_2$-$V_3$) from the first voltage difference ($V_1$-$V_2$) to determine a first CSD value ($V_1$-2$V_2$+$V_3$) associated with electrode 26-2. In a similar manner, processing module 32 may subtract first voltage difference ($V_3$-$V_4$) from first voltage difference ($V_2$-$V_3$) to determine a second CSD value ($V_2$-2$V_3$+$V_4$) associated with electrode 26-3.

Processing module 32 may then select stimulation electrodes based on the CSD values associated with electrodes 26 (106). In some examples, processing module 32 may select the electrode associated with the largest CSD value. In other examples, processing module 32 may select the electrode associated with the largest positive CSD value. In still other examples, processing module 32 may select the electrode associated with the largest negative CSD value. Processing module 32 may then control stimulation generator 30 to deliver electrical stimulation to patient 14 using the selected electrode (108).

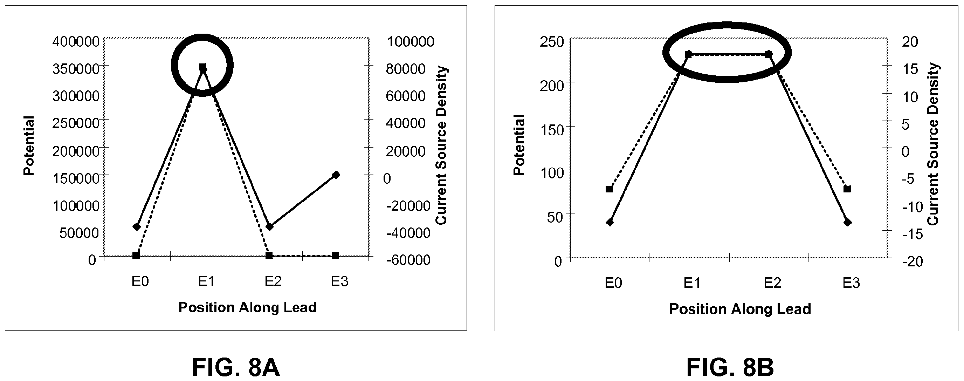

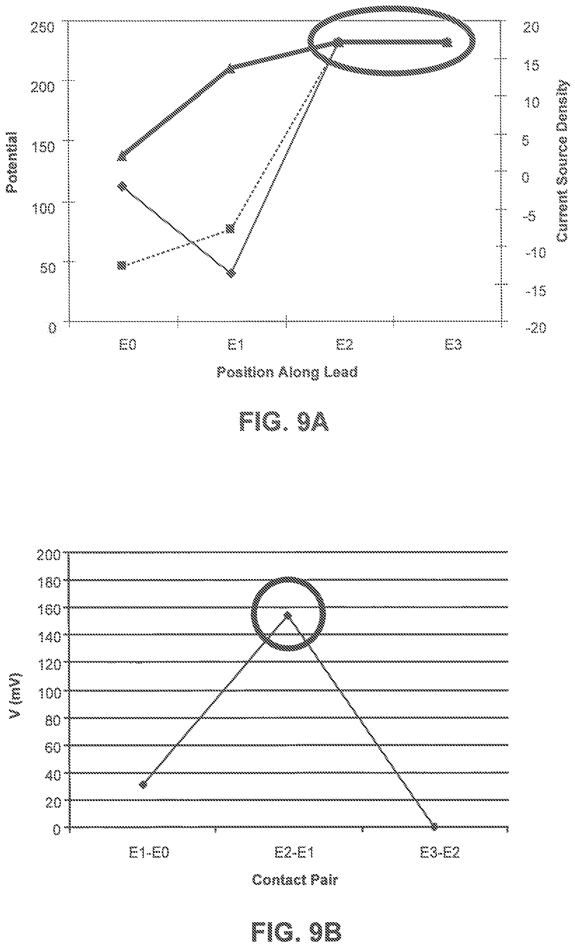

FIGS. 8A-8B and FIGS. 9A-9B show simulation results obtained by modeling electrical sensing. Briefly, extracellular potentials were generated using a point source approximation (1 mA magnitude) and the potentials were obtained at various locations on a four contact, 1.5 mm contact spacing, linear lead. The simulated results shown in FIGS. 8A-8B and FIGS. 9A-9B illustrate some advantages of CSD analysis for the selection of stimulation electrodes relative to other selection methods.

FIGS. 8A, 8B, and 9A show simulated monopolar potential measurements (dotted lines) and simulated CSD value determinations (solid lines). FIG. 9A also includes a bold line showing the absolute value of the CSD value determination. FIG. 9B shows simulated first voltage difference measurements based on the simulated potential measurements. The X-axis includes labels E0, E1, E2, and E3. E0, E1, E2, and E3 refer to four electrodes arranged in a row along a lead, e.g., E0-E4 indicate the order of electrodes from left to right along the lead. Although four electrodes are identified in the graphs of FIGS. 8A, 8B, 9A, and 9B, the simulation included six electrodes. Specifically, the simulation included one electrode to the left of E0 and one electrode to the right of E3. The two outside electrodes are not included in the graphs because CSD values were not determined for the outside electrodes. The Y-axis values are arbitrary, and may depend on the type, location, and magnitude of the modeled source.

FIGS. 8A-8B show that the determined CSD values match the monopolar potential data, which may indicate the location of the source. For example, with respect to FIG. 8A, the largest monopolar potential value was determined to be at electrode E1 and the largest CSD value was also determined to be at electrode E1. With respect to FIG. 8B, the largest monopolar potential values were determined to be at electrodes E1 and E2, as were the largest CSD values. The monopolar potential data may directly correlate with the location of the source. That is, if the source is located above a certain electrode, the electrode with the highest potential may be closest to the source. In some examples, the monopolar potential data relative to the housing electrode, or other distant electrode, may not be directly measured and used to determine CSD values because of the presence of electrical noise.

FIGS. 9A-9B illustrate an example where selecting a stimulation electrode based on CSD analysis may result in more accurate stimulation of a current source than other methods of selecting stimulation electrodes. In the example of FIGS. 9A and 9B, the model simulated a current source between electrodes E2 and E3. With reference to FIG. 9A, the largest CSD values correctly indicate that the current source was between E2 and E3. With reference to FIG. 9B, the largest voltage difference value (e.g., amongst E1-E0, E2-E1, and E3-E2) is present between electrodes E2-E1, although the current source is between electrodes E2 and E3. Accordingly, the location of a current source may be determined more accurately in some examples using CSD analysis rather than other methods, such as using the largest voltage difference between electrodes. Therefore, selecting a stimulation electrode based on CSD analysis as described in the present disclosure may provide for more accurate stimulation of current sources than other methods, such as stimulating electrodes based on the largest voltage difference between adjacent electrodes.

Equations which may be implemented by a stimulation device of the present disclosure to determine CSD values are now described. As described above, to determine a CSD value for an electrode, the stimulation device of the present disclosure may first measure voltages associated with each of the electrodes, e.g., voltages generated by polarization of neurons in the vicinity of the electrode. The stimulation device may then determine a CSD value associated with the electrodes based on the measured voltages. In some examples, the stimulation device may determine the CSD values for the electrodes by determining a second order spatial difference of the voltages along the lead. In some examples, the stimulation device may determine the CSD values based on implementation of the following equation describing the relationship between CSD and the second order spatial derivatives of the voltages:

$$\sigma \left( \frac{\partial^2 \phi}{\partial x^2} + \frac{\partial^2 \phi}{\partial y^2} + \frac{\partial^2 \phi}{\partial z^2} \right) = -C(x, y, z).$$

$$\text{(Equation 1)}$$

where $\sigma$ is the electrical conductivity of the tissue (e.g., a constant value), C is the CSD, and $\delta^2\phi/dx^2$, $\delta^2\phi/dy^2$, and $\delta^2\phi/dz^2$ are the second spatial derivatives of the voltages. In some examples, when considering a linear lead, only one component of the CSD may remain, such as $\delta^2\phi/dz^2$. Thus, the second derivative term in Equation 1 may become the second spatial difference equation, as follows:

$$D_1(z) = \frac{\phi(z + h) - 2\phi(z) + \phi(z - h)}{h^2}.$$

$$\text{(Equation 2)}$$

where h is the distance between the contacts (e.g., electrodes) at which the electrical potentials are recorded. If a lead includes 2 dimensional or 3 dimensional electrode arrays (e.g. grid electrode arrays), the stimulation device may use the sum of the second spatial differences along various dimensions to determine the CSD value for a particular electrode (Equation 1).

In some examples, using second difference values for selection of stimulation electrodes may be extended to second difference values other than second voltage differences. For example, as described hereinafter, processing module 32 may select stimulation electrodes based on second differences of other parameters associated with electrodes.

In one example, processing module 32 may determine an amount of signal content associated with each electrode within a frequency band (e.g., the beta band of 12-30 Hz or other frequency bands as listed in Table 1). For example, an amount of signal content within a frequency band may refer to a power of a signal within a frequency band.

Table 1: Frequency bands

| Frequency (f) Band Hertz (Hz) | Frequency Information |
|---|---|
| f < 5 Hz | δ (delta frequency band) |
| 5 Hz ≤ f ≤ 8 Hz | q (theta frequency band) |
| 8 Hz ≤ f ≤ 12 Hz | α (alpha frequency band) |
| 12 Hz ≤ f ≤ 16 Hz | s (sigma or low beta frequency band) |
| 16 Hz ≤ f ≤ 30 Hz | High β (high beta frequency band) |
| 50 Hz ≤ f ≤ 100 Hz | γ (gamma frequency band) |
| 100 Hz ≤ f ≤ 200 Hz | high γ (high gamma frequency band) |

Processing module 32 may assign a signal content value (e.g., power value) to each electrode indicating the amount of signal content within a frequency band. Processing module 32 may determine a second difference of the signal content values (e.g., power values) associated with the electrodes. Processing module 32 may then select an electrode to use for stimulation based on the second differences of the signal content values associated with the electrodes. For example, processing module 32 may select the electrode associated with the largest second difference value. Selection of electrodes based on the second differences of power values associated with each electrode is described hereinafter.

In some examples, with respect to lead 50 of FIG. 4, sensing module 34 may be configured to acquire electrical physiological signals via each of electrodes 52 and processing module 32 may be configured to determine an amount of signal content included within the electrical physiological signals within a frequency band. For example, sensing module 34 may sample voltages at each of electrodes 52 and processing module 32 may then perform a frequency domain analysis on the sampled signals to determine the amount of signal content (e.g., signal power) within different frequency ranges for each of electrodes 52. In some examples, processing module 32 may use a Discrete Fourier Transform (DFT) to determine the amount of signal content (e.g., power) within a selected frequency range for each of electrodes 52. In some examples, the frequency range of interest may be selected by a user. For example, a user may select a frequency range within the beta frequency band because the power within the beta frequency band (12-30 Hz) may be elevated in patient's with Parkinson's disease when they are in the off state (disease state) versus when they are in the on state (e.g., on medications).

The amount of signal content for each of electrodes 52 may be a numerical value, such as a power value. Subsequent to determining values for the amount of signal content for each of electrodes 52, processing module 32 may determine the second difference of the values in a similar manner as described above with respect to CSD values. For example, processing module 32 may first subtract the values associated with adjacent electrodes to determine first difference values. Processing module 32 may then subtract the first difference values from one another to determine second difference values. Each of the second difference values may be associated with one of electrodes 52, except for those electrodes 50-1 and 50-N.

Processing module 32 may select one or more of electrodes 52 for electrical stimulation based on the determined second difference values. For example, processing module 32 may select the electrode associated with the largest second difference value. Processing module 32 may then control stimulation generator 30 to deliver electrical stimulation using the selected electrode.

FIG. 10 is a flow diagram illustrating an example method for selecting stimulation electrodes based on second difference values associated with the electrodes. The method of FIG. 10 is described with reference to IMD 10 of FIG. 2 and lead 50 of FIG. 4. For purposes of describing the method of FIG. 10, it may be assumed that IMD 16 is configured to deliver monopolar electrical stimulation using a single one of electrodes 52 in combination with housing electrode 27.

Sensing module 32 may initially sample voltages associated with electrodes 52 (200). Processing module 32 may then determine signal content values for each of electrodes 52 (202). In some examples, processing module 32 may determine an amount of signal content (e.g., power) within a selected frequency band for each of electrodes 52.

Processing module 32 may determine first difference values between adjacent electrodes of electrodes 52 (204). Processing module 32 may then determine second difference values associated with electrodes 52 (206). Processing module 32 may select a stimulation electrode based on the second difference values associated with electrodes 52 (208). In some examples, processing module 32 may select the electrode corresponding to the largest second difference value. Processing module 32 may then control stimulation generator 32 to deliver electrical stimulation to patient 14 using the selected electrode (210).

Although the determination of CSD values is described above as being performed by an implantable medical device (e.g., IMD 16), in some examples, an external device may determine CSD values. For example, a computing device external to patient 14 (e.g., programmer 18 or a general purpose computer) may retrieve data from IMD 16 (e.g., sensing data) and determine the CSD values. Accordingly, calculation of the CSD values may be performed by IMD 16 and/or an external computing device. In examples where the CSD values are calculated by an external computing device, the external computing device may determine which electrodes to use for stimulation based on the CSD values. Subsequently, the external computing device may download the electrode selection data to IMD 16 so that IMD 16 may use the selected electrodes for stimulation.

Similarly, although the determination of a second difference of the signal content values (e.g., power values) is described above as being performed by IMD 16, in some examples, an external device may determine the second difference of the signal content values (e.g., power values). For example, a computing device external to patient 14 (e.g., programmer 18 or a general purpose computer) may retrieve data from IMD 16 (e.g., sensing data) and determine the second difference of the signal content values (e.g., power values). Accordingly, calculation of the second difference of the signal content values may be performed by IMD 16 and/or an external computing device. In examples where the second difference of the signal content values are calculated by an external computing device, the external computing device may determine which electrodes to use for stimulation based on the second difference of the signal content values. Subsequently, the external computing device may download the electrode selection data to IMD 16 so that IMD 16 may use the selected electrodes for stimulation.

The techniques described in this disclosure, including those discussed in connection with some of the Figures and those attributed to programmer, IMD, processor, and/or control circuitry, or various constituent components, may be implemented wholly or at least in part, in hardware, software, firmware or any combination thereof. A processor, as used herein, refers to any number and/or combination of a microprocessor, a digital signal processor (DSP), an application specific integrated circuit (ASIC), a field-programmable gate array (FPGA), microcontroller, processing chip, gate arrays, and/or any other equivalent integrated or discrete logic circuitry. The functions referenced herein may be embodied as firmware, hardware, software or any combination thereof as part of control circuitry specifically configured (e.g., with programming) to carry out those functions, such as in means for performing the functions referenced herein. The steps described herein may be performed by a single processing component or multiple processing components, the latter of which may be distributed amongst different coordinating devices (e.g., an IMD and an external programmer). In this way, circuitry may be distributed between multiple devices, including an implantable medical device and an external medical device in various systems. In addition, any of the described units, modules, or components may be implemented together or separately as discrete but interoperable logic devices of control circuitry. Depiction of different features as modules or units is intended to highlight different functional aspects and does not necessarily imply that such modules or units must be realized by separate hardware or software components and/or by a single device. Rather, functionality associated with one or more module or units, as part of control circuitry, may be performed by separate hardware or software components, or integrated within common or separate hardware or software components of the control circuitry.

When implemented in software, the functionality ascribed to the systems, devices and control circuitry described in this disclosure may be embodied as instructions on a physically embodied (i.e., non-transitory) computer-readable medium such as RAM, ROM, NVRAM, EEPROM, FLASH memory, magnetic data storage media, optical data storage media, or the like, the medium being physically embodied in that it is not a carrier wave, as part of control circuitry. Such medium may be removable and/or transportable (e.g., a thumb drive.) The instructions may be executed to support one or more aspects of the functionality described in this disclosure. The invention is set out in the following claims:

**Claims**

1. A system comprising:

   a stimulation generator (30) configured to generate electrical stimulation;
   a sensing module (34) configured to sense, via a plurality of electrodes (26, 28), electrical physiological signals generated by a patient; and
   a processing module (32) configured to determine current source density (CSD) values for the electrodes based on the electrical physiological signals, the processing module being configured to

   determine a power value for each of the plurality of electrodes, the power value indicating the power of the electrical physiological signals within a frequency band;
   determine second differences of the power values for the plurality of electrodes; and

control the delivery of the electrical stimulation to the patient based on the second differences of the power values; wherein

the processing module is configured to determine a plurality of first differences of the power values, each of the first differences determined based on a difference between power values of adjacent electrodes, and wherein the processing module is configured to determine the second differences based on differences between the first differences.

2. The system of claim 1, wherein the sensing module is configured to determine voltages associated with each of the plurality of electrodes, and wherein the processing module is configured to determine the power values based on the voltages determined by the sensing module.

3. The system of claim 1, wherein the processing module is configured to select one or more of the plurality of electrodes to receive the electrical stimulation based on the second differences of the power values.

4. The system of claim 3, wherein the processing module is configured to:

control the stimulation generator to deliver electrical stimulation to the selected one or more electrodes; and control the stimulation generator to refrain from delivering electrical stimulation to electrodes other than the selected one or more electrodes.

5. The system of claim 3, wherein the processing module is configured to select the one or more of the plurality of electrodes based on the magnitudes of the second differences of the power values.

6. The system of claim 5, wherein the processing module is configured to:

select the electrode having the largest second difference value; control the stimulation generator to deliver electrical stimulation to the electrode having the largest second difference value; and control the stimulation generator to refrain from delivering electrical stimulation to electrodes other than the electrode having the largest second difference value.

7. The system of claim 1, wherein the processing module is configured to apportion the electrical stimulation between the plurality of electrodes based on the second differences of the power values.

8. A non-transitory storage medium storing instructions which, when run on a system of any of the preceeding claims, cause the processing module to:

receive an indication of sensed electrical physiological signals sensed from a patient via a plurality of electrodes and determine current source density values (CSD) for the electrodes based on the sensed signals; the instructions causing the processing module to determine the current source density values by:

determining a power value for each of the plurality of electrodes, the power value indicating the power of the electrical physiological signals within a frequency band; determining a plurality of first differences of the power values, each of the first differences determined based on a difference between power values of adjacent electrodes; determining second differences of the power values for the plurality of electrodes;

the processing module is configured to determine the second differences based on differences between the first differences; and to control the delivery of the electrical stimulation to the patient based on the second differences of the power values.

**Patentansprüche**

1. System, das Folgendes enthält:

einen Stimulationsgenerator (30), der konfiguriert ist, eine elektrische Stimulation zu erzeugen;

ein Erfassungsmodul (34), das konfiguriert ist, über mehrere Elektroden (26, 28) elektrische physiologische Signale, die durch einen Patienten erzeugt werden, zu erfassen; und

ein Verarbeitungsmodul (32), das konfiguriert ist, Stromquellendichtewerte (CSD-Werte) für die Elektroden anhand des elektrischen physiologischen Signals zu bestimmen, wobei das Verarbeitungsmodul konfiguriert ist,

einen Leistungswert für jede der mehreren Elektroden zu bestimmen, wobei der Leistungswert die Leistung für die elektrischen physiologischen Signale innerhalb eines Frequenzbandes angibt;

zweite Differenzen der Leistungswerte für die mehreren Elektroden zu bestimmen; und

die Verabreichung der elektrischen Stimulation an den Patienten anhand der zweiten Differenzen der Werte zu steuern; wobei

das Verarbeitungsmodul konfiguriert ist, mehrere erste Differenzen der Leistungswerte zu bestimmen, wobei jede der ersten Differenzen auf einer Differenz zwischen Leistungswerten benachbarter Elektroden beruht, und wobei

das Verarbeitungsmodul konfiguriert ist, die zweiten Differenzen anhand von Differenzen zwischen den ersten Differenzen zu bestimmen.

2. System nach Anspruch 1, wobei das Erfassungsmodul konfiguriert ist, Spannungen, die jeder der mehreren Elektroden zugeordnet sind, zu bestimmen, und wobei das Verarbeitungsmodul konfiguriert ist, die Leistungswerte anhand der durch das Erfassungsmodul bestimmten Spannungen zu bestimmen.

3. System nach Anspruch 1, wobei das Verarbeitungsmodul konfiguriert ist, anhand der zweiten Differenzen der Leistungswerte eine oder mehrere der mehreren Elektroden auszuwählen, um die elektrische Stimulation zu empfangen.

4. System nach Anspruch 3, wobei das Verarbeitungsmodul konfiguriert ist:

den Stimulationsgenerator zu steuern, um eine elektrische Stimulation an die eine oder die mehreren ausgewählten Elektroden zu liefern;

den Stimulationsgenerator zu steuern, die Lieferung einer elektrischen Stimulation an Elektroden, die von der einen oder den mehreren ausgewählten Elektroden verschieden sind, zu unterlassen.

5. System nach Anspruch 3, wobei das Verarbeitungsmodul konfiguriert ist, die eine oder die mehreren der mehreren Elektroden anhand der Größen der zweiten Differenzen der Leistungswerte auszuwählen.

6. System nach Anspruch 5, wobei das Verarbeitungsmodul konfiguriert ist:

die Elektrode mit dem größten zweiten Differenzwert auszuwählen;

den Stimulationsgenerator zu steuern, um eine elektrische Stimulation an die Elektrode mit dem größten zweiten Differenzwert zu liefern; und

den Stimulationsgenerator zu steuern, die Lieferung einer elektrischen Stimulation an Elektroden, die von der Elektrode mit dem größten zweiten Differenzwert verschieden sind, zu unterlassen.

7. System nach Anspruch 1, wobei das Verarbeitungsmodul konfiguriert ist, die elektrische Stimulation zwischen den mehreren Elektroden anhand der zweiten Differenzen der Leistungswerte aufzuteilen.

8. Nichtflüchtiges Speichermedium, das Befehle speichert, die dann, wenn sie auf einem System nach einem der vorhergehenden Ansprüche abgearbeitet werden, das Verarbeitungsmodul dazu veranlassen:

eine Angabe erfasster elektrischer physiologischer Signale, die von einem Patienten erfasst werden, über mehrere Elektroden zu empfangen und Stromquellendichtewerte (CSD-Werte) für die Elektroden anhand der erfassten Signale zu bestimmen;

wobei die Befehle das Verarbeitungsmodul dazu veranlassen, die Stromquellendichtewerte zu bestimmen durch:

Bestimmen eines Leistungswertes für jede der mehreren Elektroden, wobei der Leistungswert die Leistung der elektrischen physiologischen Signale innerhalb eines Frequenzbandes angibt;

Bestimmen mehrerer erster Differenzen der Leistungswerte, wobei jede der bestimmten ersten Differenzen auf einer Differenz zwischen Leistungswerten benachbarter Elektroden beruht;

Bestimmen zweiter Differenzen der Leistungswerte für die mehreren Elektroden;

wobei das Verarbeitungsmodul konfiguriert ist, die zweiten Differenzen anhand von Differenzen zwischen den ersten Differenzen zu bestimmen; und
Steuern der Verabreichung der elektrischen Stimulation an den Patienten anhand der zweiten Differenzen der Leistungswerte.

**Revendications**

1. Système comportant :

   un générateur de stimulation (30) configuré pour générer une stimulation électrique ;
   un module de détection (34) configuré pour détecter, via une pluralité d'électrodes (26, 28,) des signaux physiologiques électriques générés par un patient ; et
   un module de traitement (32) configuré pour déterminer des valeurs de densité de source de courant (CSD) pour les électrodes sur la base des signaux physiologiques électriques, le module de traitement étant configuré pour :

      déterminer une valeur de puissance pour chaque électrode de la pluralité d'électrodes, la valeur de puissance indiquant la puissance des signaux physiologiques électriques sur une bande de fréquences ;
      déterminer des secondes différences des valeurs de puissance pour la pluralité d'électrodes ; et
      commander la délivrance de la stimulation électrique au patient sur la base des secondes différences des valeurs de puissance ; dans lequel

   le module de traitement est configuré pour déterminer une pluralité de premières différences des valeurs de puissance, chacune des premières différences étant déterminée sur la base d'une différence entre des valeurs de puissance d'électrodes adjacentes, et dans lequel
   le module de traitement est configuré pour déterminer les secondes différences sur la base de différences entre les premières différences.

2. Système selon la revendication 1, dans lequel le module de détection est configuré pour déterminer des tensions associées à chaque électrode de la pluralité d'électrodes, et dans lequel le module de traitement est configuré pour déterminer les valeurs de puissance sur la base des tensions déterminées par le module de détection.

3. Système selon la revendication 1, dans lequel le module de traitement est configuré pour sélectionner une ou plusieurs électrodes parmi la pluralité d'électrodes pour recevoir la stimulation électrique sur la base des secondes différences des valeurs de puissance.

4. Système selon la revendication 3, dans lequel le module de traitement est configuré pour :

   commander le générateur de stimulation de manière à délivrer une stimulation électrique à la ou aux électrodes sélectionnées ; et
   commander le générateur de stimulation de manière à s'abstenir de délivrer la stimulation électrique à des électrodes autres que la ou les électrodes sélectionnées.

5. Système selon la revendication 3, dans lequel le module de traitement est configuré pour sélectionner la ou les électrodes parmi la pluralité d'électrodes sur la base des grandeurs des secondes différences des valeurs de puissance.

6. Système selon la revendication 5, dans lequel le module de traitement est configuré pour :

   sélectionner l'électrode ayant la seconde plus grande valeur de différence ;
   commander le générateur de stimulation pour délivrer la stimulation électrique à l'électrode ayant la seconde plus grande valeur de différence ; et
   commander le générateur de stimulation pour s'abstenir de délivrer la stimulation électrique à des électrodes autres que l'électrode ayant la seconde plus grande valeur de différence.

7. Système selon la revendication 1, dans lequel le module de traitement est configuré pour répartir la stimulation électrique entre la pluralité d'électrodes sur la base des secondes différences des valeurs de puissance.

8. Support de stockage non transitoire stockant des instructions qui, lorsqu'elles sont exécutées sur un système selon l'une quelconque des revendications précédentes, amènent le module de traitement à :

recevoir une indication de signaux physiologiques électriques détectés provenant d'un patient via une pluralité d'électrodes et déterminer des valeurs de densité de source de courant (CSD) pour les électrodes sur la base des signaux détectés ;
les instructions amenant le module de traitement à déterminer les valeurs de densité de source de courant :

en déterminant une valeur de puissance pour chaque électrode de la pluralité d'électrodes, la valeur de puissance indiquant la puissance des signaux physiologiques électriques sur une bande de fréquences ;
en déterminant une pluralité de premières différences des valeurs de puissance, chacune des premières différences étant déterminée sur la base d'une différence entre des valeurs de puissance d'électrodes adjacentes ;
en déterminant des secondes différences des valeurs de puissance pour la pluralité d'électrodes ;

le module de traitement est configuré pour déterminer les secondes différences sur la base de différences entre les premières différences ; et pour commander la délivrance de la stimulation électrique au patient sur la base des secondes différences des valeurs de puissance.

**FIG. 1**

**FIG. 2**

| | Electrode Voltages | First Voltage Differences | CSD Values |
|---|---|---|---|
| 26-1 | $V_1$ | | |
| | | $V_1 - V_2$ | |
| 26-2 | $V_2$ | | $CSD_1$ $V_1 - 2V_2 + V_3$ |
| | | $V_2 - V_3$ | |
| 26-3 | $V_3$ | | $CSD_2$ $V_2 - 2V_3 + V_4$ |
| | | $V_3 - V_4$ | |
| 26-4 | $V_4$ | | |

**FIG. 3**

**FIG. 4**

**FIG. 5A**

**FIG. 5B**

EP 2 844 336 B1

60

62

**FIG. 6A**

60

64-1    64-2    64-3    64-4    64-5    64-6    64-7    64-8

**FIG. 6B**

80

66-6

66-5

66-4

66-3

66-2

66-1

68    70

**FIG. 6C**

FIG. 7

FIG. 8B

FIG. 8A

FIG. 9A

FIG. 9B

FIG. 10

**EP 2 844 336 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110264165 A

- WO 2010044989 A